# EUROPEAN PATENT SPECIFICATION

(11) **EP 2 029 026 B1**
(45) Date of publication and mention of the grant of the patent: **02.12.2015**
(21) Application number: 07722735.3
(22) Date of filing: 07.06.2007
(51) Int. Cl.: A61B 10/02

(54) **A DEVICE FOR COLLECTING SAMPLES**
VORRICHTUNG ZUR ENTNAHME VON PROBEN
APPAREIL POUR PRÉLEVER DES ÉCHANTILLONS

(30) Priority: 09.06.2006 EE 200600019
(43) Date of publication of application: 04.03.2009
(73) Proprietor: Eesti Maaülikool, 51014 Tartu (EE)
(72) Inventor: VALDMANN, Andres, EE51014 Tartu (EE); VALDMANN, Merle, EE51014 Tartu (EE)
(74) Representative: Kahu, Sirje
(86) International application number: PCT/EE2007/000011
(87) International publication number: WO 2007/140792

(56) References cited:
- US-A- 4 243 048
- US-A1- 2005 277 846
- US-A1- 2005 288 606

## Description

### TECHNICAL FIELD OF INVENTION

The invention belongs to the veterinary field and will be applied to collect cytological, bacteriological and uterine secretion samples primarily from the uterine endometrium with the aim of detecting inflammations, including subclinical endometritis.

### BACKGROUND OF THE INVENTION

To detect the inflammations, the presence and counts of white blood cells and bacteria in cytological and bacteriological material and presence and quantity of inflammation markers in uterine secretion are determined. On the basis of the presence and count of white blood cells and bacteria and inflammation markers it is possible to diagnose uterine inflammations.

The maximum diagnostic precision is guaranteed by the cleanness of the material. The collection of pure material is more difficult in animal species (e.g. cows) whose uterine cervix is difficult to penetrate due to the anatomical build and/or the physiological status of the animal. The use of an unsuitable device to penetrate the uterine cervix may damage it, creating favourable conditions for inflammation; and excessive manipulation of the device (by moving it back and forth) creates the risk of its contamination, which leads to the impossibility of collecting clean material, and this may affect the analysis result and diagnostic precision. Another important problem is the collecting of clean material from animals with a high body weight. Even the smallest movement of an animal with a high body weight may cause the contamination of the device during sample collection.

Numerous devices have been created for the collection of cytological and bacteriological material, with the help of which it is possible to collect material from the uterus, and which are intended for use in women.

A device for collecting uterine cervical mucosa samples is known, which consists of a Cytobrush with a conical distal penetrating spike, placed in a cylindrical sleeve (US6336905, A61B10/00, Colaianni Rana, 2002). The endometrial tissue sample brush, which consists of a flexible rod with distal core tip, placed in a flexible sheath, cylindrical brush and antitrauma cap, is described in a US patent (US5713368, A61B10/00, Medical Device Technologies, Inc., 1998). An endometrium sample collection brush, which contains a sliding flexible sleeve for brush protection, is also known (US4227537, A61B10/00, Tucson Medical Instruments Inc., 1980).

Of devices designed for animals, a device for collecting cells and bacteriological samples is known which consists of a flexible tube with a rod in it which has peripheral grooves for cell samples at the tip and moisture absorbing material, a swab, close to it for bacteriological sample (EP0087402, A61B10/00, Nydahl Claes, 1983).

Another veterinary device for culture collection is known from US 2005/0288606.

The closest technical solution is a device which is designed for collecting material from animals and consists of a tube with a rod placed in it which has a brush or swab attached to collect cytological or bacteriological material (Can J Vet., 2005, 46, 255-259).

The shortcomings of this device when penetrating the uterine cervix are the possibility of uterine cervical damage, which creates favourable conditions for inflammation; and the risk of device contamination when it is extensively manipulated (moved back and forth), which makes it impossible to collect a clean sample and may influence the analysis result.

### DISCLOSURE OF THE INVENTION

The aim of this invention is to introduce a device for collecting cytological, bacteriological and uterine secretion samples, including clean diagnostic material samples, consisting of a guiding tube and a rod extending through the guiding tube with a brush attached, and containing in addition a guiding tube cap that can be attached to a guiding tube with an inwards-facing cone, a Cytobrush ball and a rod-fixating screw located on the rear end of the guiding tube.

The Cytobrush is connected to the rod by a semi-rigid fastening tube.

The Cytobrush guiding tube cap has a smaller diameter than the Cytobrush guiding tube to improve uterine cervical penetration. Transition from the guiding tube cap to the guiding tube is smooth. The Cytobrush ball, together with the guiding tube cap, forms the smooth transition to the guiding tube.

To avoid the contamination of the Cytobrush, the guiding tube cap is equipped with an inward-facing cone so that the Cytobrush ball fits tightly inside the guiding tube cap.

The inner diameter of the Cytobrush guiding tube cap is designed so that only the Cytobrush fits into it, but not the Cytobrush fixating rod. Such a design makes it possible to advance the Cytobrush from the guiding tube cap an optimal distance and avoid the Cytobrush penetrating too far into the uterus. Such a design guarantees sample collection precisely from the desired area.

### BRIEF DESCRIPTION OF THE DRAWINGS

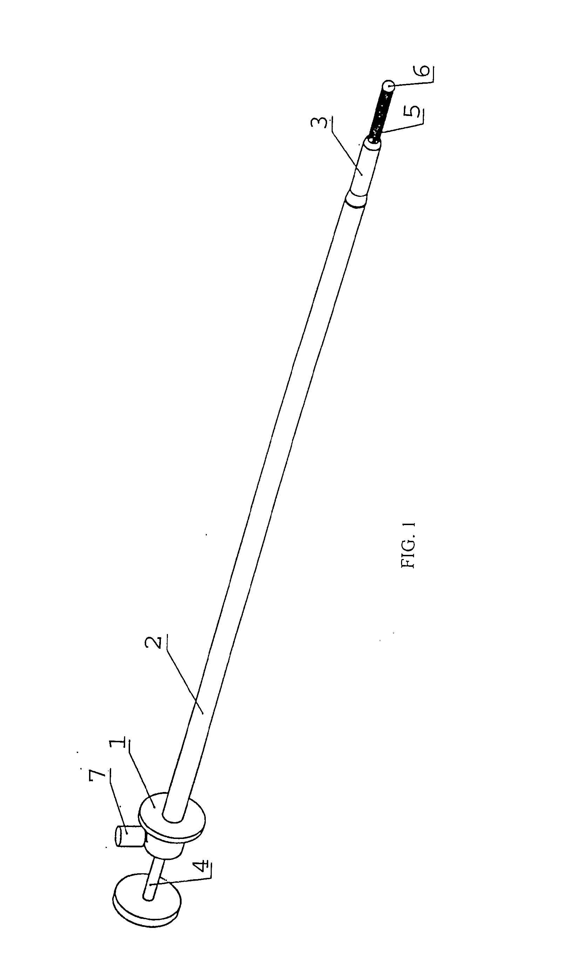

Figure 1 shows the overview of the sample collection device, and

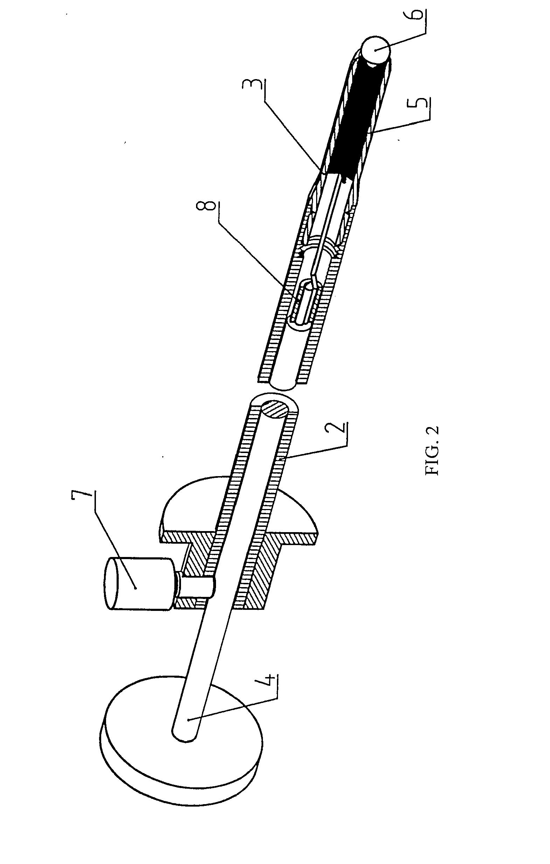

Figure 2 shows the longitudinal section of the device shown on Figure 1.

### DESCRIPTION OF EMBODIMENTS

Before sample collection, the device 1 was sterilized. Before the application of the device, the device 1 was set in its working position by unfastening the rod-fixating screw 7. The Cytobrush was retracted into the guiding tube cap 3 with the help of the device's rod 4. The Cytobrush was fixated with the rod-fixating screw 7 and the device 1 was then introduced into the uterus via the vagina and uterine cervix. When the device 1 was introduced into the desired region, the rod-fixating screw 7 was unfastened and the rod was pushed into the guiding tube 2, as a result of which the Cytobrush advanced into the uterus, where it contacted with the endometrium. By rotating the rod 4 multiple times around its axle, endometrium material was collected on the Cytobrush 5.

Then the Cytobrush was retracted into the guiding cap 3 with the help of rod 4. The rod 4 was fixated with the help of the rod-fixating screw 7 and the device 1 was taken out of the uterus and the animal. The fixation of the rod by the screw 7 allows the withdrawal of the device safely from the uterus (without contamination of Cytobrush by material from the uterine cervix or vagina).

To remove the material from the Cytobrush the rod-fixating screw 7 was unfastened, the Cytobrush 5 was advanced from the guiding tube cap 3 by pushing the rod 4 into the guiding tube and then the rod was fixated with the rod-fixating screw 7. To obtain bacteriological material, the Cytobrush ball 6 was cut off with scissors, and a piece was cut from the Cytobrush and placed into a transport medium together with the obtained material. To obtain uterine secretion, the Cytobrush ball 6 was cut off with scissors, and a piece was cut from the Cytobrush and placed into a transport buffer together with the obtained uterine secretion. To separate the cytological sample material from the Cytobrush 5, the Cytobrush 5 was rolled over a glass microscope slide, and the sample was then fixed.

## Claims

1. A device for collecting cytological, bacteriological and uterine secretion samples comprising a guiding tube (2), a rod (4) extending through the guiding tube with a Cytobrush (5) attached to said rod a rod-fixating screw (7) located on the rear end of the guiding tube,
**characterized in that** the guiding tube (2) is rigid and the device further comprises
a) a cytobrush ball (6) attached to said Cytobrush (5), wherein said Cytobrush (5) is connected to the rod (4) by a semi-rigid fastening tube (8), and
b) a guiding tube cap (3) attached to the guiding tube wherein said cap has an inward-facing cone so that the cytobrush ball (6) fits tightly inside the cap to avoid the contamination of the Cytobrush (5) and obtain clean diagnostic material,
and to improve uterine cervical penetration and to achieve smooth transition from the guiding tube cap (3) to the guiding tube (2), the guiding tube cap (3) has a smaller diameter than the guiding tube (2),
and to obtain diagnostic material precisely from the desired area, and to advance the Cytobrush from the guiding tube cap (3) to an optimal distance and to avoid the Cytobrush (5) penetrating too far into the uterus, the inner diameter of the guiding tube cap (3) is smaller than the diameter of the rod (4).

## Patentansprüche

1. Vorrichtung zur Entnahme von zytologischen, bakteriologischen und uterinen Sekretproben, die ein Leitrohr (2), eine Stange (4), die sich zusammen mit einer Zytobürste (5), die der genannten Stange hinzugefügt worden ist, durch das Leitrohr erstreckt, eine Schraube (7) zum Fixieren der Stange, wobei die Schraube sich am rückwärtigen Ende des Leitrohrs befindet, umfasst,
**dadurch gekennzeichnet, dass** das Leitrohr (2) unbiegsam ist und dass die Vorrichtung zusätzlich folgendes umfasst:
a) eine Kugel der Zytobürste (6), die der genannten Zytobürste (5) hinzugefügt worden ist, wobei die genannte Zytobürste (5) durch ein halbstarres Befestigungsrohr (8) mit der Stange (4) verbunden ist; und
b) eine Rohrkappe (3), die dem Leitrohr hinzugefügt worden ist, wobei die genannte Rohrkappe einen nach innen gerichteten Konus hat, so dass die Kugel der Zytobürste (6) fest in der Rohrkappe sitzt, um Kontaminierung der Zytobürste (5) zu vermeiden und sauberes Probenmaterial zu erhalten,
und um die Penetration des Gebärmutterhalses zu verbessern und einen reibungslosen Übergang aus der Rohrkappe (3) des Leitrohrs in den Leitrohr (2) zu erreichen, hat die Rohrkappe (3) des Leitrohrs einen kleineren Durchmesser als das Leitrohr (2),
und um Probenmaterial genau aus dem gewünschten Bereich zu erhalten, und um die Zytobürste in eine optimale Entfernung von der Rohrkappe (3) des Leitrohrs zu befördern und um eine zu weite Penetration der Zytobürste (5) in den Uterus hinein zu vermeiden, ist das Innendurchmesser der Rohrkappe (3) des Leitrohrs kleiner als das Durchmesser der Stange (4).

## Revendications

1. Appareil pour prélever des échantillons de sécrétion cytologiques, bactériologiques et de l'utérus, comprenant un tube de guidage (2), une tige (4) traversant le tube de guidage avec la cytobrosse (5) fixée à ladite tige, une vis de fixation de la tige (7) située sur l'extrémité arrière du tube de guidage,
**caractérisé en ce que** le tube de guidage (2) est rigide et le appareil comprend en outre :
a) une bille de cytobrosse (6) fixée á ladite cytobrosse (5), et ladite cytobrosse (5) est reliée á la tige (4) par un tube de fixation semi-rigide (8) et
b) un capuchon de tube de guidage (3) fixé au tube de guidage, et ledit capuchon comporte un cône tourné vers l'intérieur de sorte que la bille de cytobrosse (6) soit logée de façon adapte étroitement à l'intérieur du capuchon pour éviter la contamination de la cyrobrosse (5), nécessaire pour obtenir du matièrel de diagnostic propre,
et pour améliorer la pénétration dans le col de l'utérus, ainsi que pour assurer le passage en douceur à partir de capuchon du tube de guidage (3) vers le tube de guidage (2), le capuchon du tube de guidage (3) ayant un diamètre inférieur au tube de guidage (2), et pour obtenir de la matière de diagnostic avec l'adresse dans la zone voulue, et pour faire avancer le cytobrosse capuchon de tube de guidage (3) à une distance optimale, et pour éviter la pénétration de la cytobrosse (5) trop avancée dans l'utérus, le diamètre intérieur du capuchon du tube de guidage (3) est inférieur au diamètre de la tige (4).
